Europäisches Patentamt

European Patent Office

Office européen des brevets

(11) Publication number: **0 327 382**
**A2**

## (12) EUROPEAN PATENT APPLICATION

(21) Application number: **89301065.2**

(22) Date of filing: **03.02.89**

(51) Int. Cl.⁴: **A 61 K 7/02**
**A 61 K 7/48, C 09 K 3/00**

(30) Priority: **05.02.88 GB 8802614**

(43) Date of publication of application:
**09.08.89 Bulletin 89/32**

(84) Designated Contracting States:
**AT BE CH DE ES FR GR IT LI LU NL SE**

(71) Applicant: **ECC INTERNATIONAL LIMITED**
**John Keay House**
**St. Austell Cornwall PL25 4DJ (GB)**

(72) Inventor: **Smith, Douglas Neil**
**8 Polyear Close Polgooth**
**St. Austell Cornwall (GB)**

**Goodman, Howard**
**40 Turnavean Road**
**St. Austell Cornwall (GB)**

**Buse, William Roger**
**8 Trelyn Rock**
**Wadebridge Cornwall (GB)**

(74) Representative: **Bull, Michael Alan et al**
**Haseltine Lake & Co. Hazlitt House 28 Southampton**
**Buildings Chancery Lane**
**London WC2A 1AT (GB)**

The title of the invention has been amended (Guidelines for Examination in the EPO, A-III, 7.3).

(54) **Thixotropic composition containing an alcohol and water.**

(57) There is disclosed a stable, thixotropic, fluid composition which comprises water, a lower aliphatic alcohol, a hydrophilic smectite clay and a relatively low molecular weight hydrophilic polymer, wherein the ratio by weight of lower aliphatic alcohol to water is in the range 1:4 to 7:3, the ratio by weight of clay to water is in the range of from 1:4 to 1:19; and the ratio by weight of hydrophilic polymer to smectite clay is in the range of from 0.05:1 to 0.5:1.

Also disclosed is a method of making the composition.

The composition may be used in the formulation of lotions and creams for personal care and toiletry products.

**Description**

## COMPOSITION CONTAINING AN ALCOHOL AND WATER

This invention relates to compositions containing a lower aliphatic alcohol and water, and to a method for thickening mixtures consisting predominantly of a lower aliphatic alcohol, i.e. one having from one to four carbon atoms, and water to form compositions which can be used, for example, as or in the preparation of personal care and toiletry creams and lotions, especially hand cleansing creams.

## BACKGROUND OF THE INVENTION

Hitherto mixtures of lower aliphatic alcohols and water have usually been thickened by incorporating therein a hydrophilic organic polymer such as hydroxyethyl cellulose. However the resulting compositions are generally found to have two serious disadvantages: firstly, the viscosifying effect of the polymer is generally sensitive to temperature so that when the temperature of the composition is raised above about 30°C the gel structure in the composition is weakened leaving the composition undesirably fluid; and secondly, when the composition is dispensed by forcing it under pressure through a narrow orifice again the viscosity of the composition is reduced giving a watery consistency and the gel structure is regained only slowly over a period of time, this phenomenon being known as "slow thixotropy".

Attempts have been made to use mineral materials as thickening agents for alcohol-water mixtures but these do not generally disperse well in alcohol-rich media with the result that either the composition has a gritty texture or the mineral material is partially flocculated to form discrete flocs separated by visible expanses of clear liquid. These mineral thickening agents also tend to settle out on prolonged storage.

## THE PRIOR ART

GB-A-2179336 describes a liquid composition containing a significant quantity of a lower aliphatic alcohol which is thickened by adding thereto from 1% to 10% by weight, based on the weight of the aliphatic alcohol, of an organo clay which has been prepared by mixing with a smectite clay a mixture of two particular quaternary ammonium compounds and by subjecting the resultant composition to high shear mixing. The thickening agents described in this published patent application are especially suitable for use with alcohol-water mixtures containing 90% by weight or more of alcohol.

GB-A-1599352 relates to a thixotropic aqueous coating composition or latex-type paint. The composition comprises a film-forming polymer, finely divided silica, an alcohol of molecular weight from 32 to 118, a cationic surfactant and water together with a gel formed from an amine-modified smectite clay and a water-immiscible liquid hydrocarbon. The composition is stated to exhibit the desirable film-forming and application properties of a latex type coating, yet which will not leave insoluble residues on spraying equipment following evaporation of the volatile components.

DE-A-2951319 relates to a polymeric sealing compound for medical purposes. Initially, a paste is formed by combining in a solvent (a mixture of water and alcohol), polymers and gel-forming substances, predominantly comprising karaya gum but optionally also including an inorganic neutral gel former such as an organic montmorillonite derivative. The solvent predominantly comprises alcohol (in the range of from 75-93% by weight of the total solvent in the Examples). It is believed that an organic montmorillonite (which will be organophilic in character) can be chosen as the inorganic gel forming substance in view of the very high relative quantity of alcohol in the solvent (also see GB-2179336 referred to above). The paste, which is mouldable, is applied to a surface region of a body and allowed to harden by evaporation of the solvent.

DE-A-2551891 relates to a mouldable self-supporting (or free-standing) gel that is made up from water, a synthetic montmorillonite clay, and first and second polymerisates. An aqueous pre-mix of each polymerisate is prepared, one of which pre-mixes also includes the clay. The pre-mixes are combined and poured into moulds to set. During the setting period it is believed that the two polymerisates cross-link to form the rigid self-supporting gel. In addition, the pre-mixes may also be combined with up to 30% by weight of a water soluble alcohol before moulding. The solid gels find use, for example, in air fresheners, and water beds.

## THE INVENTION

According to a first aspect of the present invention there is provided a stable, thixotropic, fluid composition which comprises water, a lower aliphatic alcohol, a hydrophilic smectite clay and a relatively low molecular weight hydrophilic polymer, wherein the ratio by weight of lower aliphatic alcohol to water is in the range of from 1:4 to 7:3, the ratio by weight of clay to water is in the range of from 1:4 to 1:19; and the ratio by weight of hydrophilic polymer to smectite clay is in the range of from 0.05:1 to 0.5:1.

According to a second aspect of the present invention there is provided a method of preparing a stable, thixotropic, fluid composition which consists of a mixture of a lower aliphatic alcohol and water in relative amounts of from 1:4 to 7:3, the method comprising the following steps:

a) mixing a hydrophilic smectite clay mineral with water to form a viscous suspension, the ratio by weight of clay to water being in the range of from 1:4 to 1:19;

b) mixing with the viscous suspension a hydrophilic polymer of relatively low molecular weight in a quantity such that the weight ratio of hydrophilic polymer to dry smectite clay mineral is in the range of from 0.05:1 to 0.5:1; and

2

c) mixing with the suspension formed in step b) a lower aliphatic alcohol.

The compositions of the invention may be used, as or in the preparation of, personal care and toiletry creams and lotions.

According to a third aspect of the present invention, there is provided a creamy base composition for use as, or in the preparation of, personal care and toiletry creams and lotions, which comprises water, a lower aliphatic alcohol, a hydrophilic smectite clay and a relatively low molecular weight hydrophilic polymer, wherein the ratio by weight of lower aliphatic alcohol to water is in the range 1:4 to 7:3, the ratio by weight of clay to water is in the range of from 1:4 to 1:19; and the ratio by weight of hydrophilic polymer to smectite clay is in the range of from 0.05:1 to 0.5:1

## DETAILED DESCRIPTION

The compositions of the invention have a gel structure which is substantially stable at temperatures up to about 40°C and which recovers rapidly after extrusion through a narrow office. The compositions of the invention are creamy in texture and are fluid. They therefore find utility as bases for lotions and creams. The compositions are not rigid or selfsupporting.

In step a), the hydrophilic smectite clay may be bentonite, montmorillonite, saponite or the like. The quantity of the smectite clay used is preferably in the range of from 5% to 20% by weight of the clay-water mixture. By the term hydrophilic it is meant that the clay has not been treated with a quaternary ammonium compound having at least one higher alkyl radical which would render the clay hydrophilic and organophilic. The smectite clay is preferably the only gel-forming substance used.

In step b), the hydrophilic polymer is preferably sodium carboxymethyl cellulose, and the weight ratio of the sodium carboxymethyl cellulose to the dry smectite clay is preferably in the range of from 0.1:1 to 0.5:1, more preferably 0.1:1 to 0.4:1. A hydrophilic polymer is considered to be of "relatively low molecular weight" if a 2% by weight suspension of the polymer in water is found to have a viscosity of less than 100 mPas at 22°C, as measured by a Brookfield Viscometer with a spindle speed of 60 rpm. It will be found that usually the compositions of the invention will contain from about 0.1% to 2.0% by weight of the hydrophilic polymer, based on the weight of the composition.

In step c), after admixing the lower aliphatic alcohol with the suspension containing the smectite clay, water and hydrophilic polymer, it is advantageous to subject the mixture to high shear mixing. By lower aliphatic alcohol is meant one having from 1 to 4 carbon atoms.

The method of the present invention is particularly suitable for use when the amount of alcohol added in step c) is such that the alcohol constitutes from about 20% to about 70%, preferably 50 to 70%, of the total weight of alcohol and water in the composition.

The composition of the invention can be used as such or there can be incorporated therein conventional additives employed in the manufacture of personal care and toiletry creams and lotions.

Other than conventional additives required in the making up of personal care and toiletry creams and lotions, the compositions of the invention may consist essentially only of the ingredients recited.

The invention is illustrated by the following Examples.

## EXAMPLE 1

A viscous suspension was prepared by mixing 16g of a bentonite (which had a particle size distribution such that 99% by weight passed through a No. 300 mesh British Standard sieve - nominal aperture 53 microns -and a cation exchange capacity of 80 meg/100g of dry bentonite) with 194g of water by means of a paddle stirrer rotating at 1000 rpm for 15 minutes. To 25g of this 8% by weight suspension of bentonite there were then added, with stirring, 5g of water and 0.67g of a sodium carboxymethyl cellulose which was sold under the trade mark "FINNFIX 5" and which was found to have a viscosity in a 2% by weight aqueous suspension at 22°C of less than 100 mPas as measured by a Brookfield Viscometer with a spindle speed of 60 rpm. To this mixture there was then added with stirring, 54g of isopropanol. The resulting composition was finally subjected to high shear mixing using a "SILVERSON" shrouded impeller mixer for 1 minute.

The final composition contained 63.8% by weight of isopropanol and the weight ratio of sodium carboxymethyl cellulose to dry bentonite was 0.33:1. The viscosity of the final composition was then measured using a Brookfield Viscometer with spindle no. 3 rotating at 100 rpm at temperatures of 21°C and 36°C respectively. The results obtained are set forth in Table I below.

## EXAMPLE 2

The experiment described in Example 1 was repeated except that the hydrophilic polymer was replaced by an identical quantity of a different sodium carboxymethyl cellulose which was sold under the trade mark "BLANOSE 7L3". This polymer also had a viscosity in a 2% by weight aqueous suspension at 22°C of less than 100 mPas when measured by a Brookfield Viscometer with a spindle speed of 60 rpm.

## EXAMPLE 3

The experiment described in Example 1 was repeated except that the bentonite was replaced by an identical quantity of a different bentonite which had a particle size distribution such that 80% by weight passed through a No. 200 mesh British Standard sieve (nominal aperture 76 microns) and a cation exchange capacity of 100 meq/100g of dry bentonite.

## EXAMPLE 4

The experiment described in Example 1 was repeated except that the final suspension contained 30g of an aqueous suspension containing 10% by weight of the bentonite described in Example 1, 0.50g of the sodium carboxymethyl cellulose described in Example 1 and 54g of isopropanol. The final composition thus contained 63.9% by weight of isopropanol and the weight ratio of sodium carboxymethyl cellulose to dry bentonite was 0.17:1.

## EXAMPLE 5

The experiment described in Example 1 was repeated except that the final suspension contained 27g of an aqueous suspension containing 15% by weight of the bentonite described in Example 1, 1.00g of the sodium carboxymethyl cellulose described in Example 1, 2.5g of water and 54g of isopropanol. The final composition thus contained 63.9% of by weight of isopropanol and the weight ratio of sodium carboxymethyl cellulose to dry bentonite was 0.25:1.

## EXAMPLE 6

The experiment described in Example 1 was repeated except that the final suspension contained 25g of an aqueous suspension containing 8% by weight of the bentonite described in Example 1, 0.67g of the sodium carboxymethyl cellulose described in Example 1, 5g of water and 69g of isopropanol. The final composition thus contained 69% by weight of isopropanol and the weight ratio of sodium carboxymethyl cellulose to dry bentonite was 0.33:1.

## EXAMPLE 7

The experiment of Example 6 was repeated except that the bentonite was replaced by an identical quantity of the bentonite which was used in Example 3.

## EXAMPLE 8

The experiment described in Example 1 was repeated except that the final suspension contained 26.67g of an aqueous suspension containing 15% by weight of the bentonite described in Example 1, 1.33g of the sodium carboxymethyl cellulose described in Example 1, 6g of water and 66g of isopropanol. The final composition thus contained 66% by weight of isopropanol and the weight ratio of sodium carboxymethyl cellulose to dry bentonite was 0.33:1.

TABLE 1

| Example No. | % by weight of isopropanol | weight ratio polymer: bentonite | Viscosity (mPas) | |
|---|---|---|---|---|
| | | | 21°C | 36°C |
| 1 | 63.8 | 0.33 | 620 | 585 |
| 2 | 63.8 | 0.33 | 750 | 620 |
| 3 | 63.8 | 0.33 | 365 | 480 |
| 4 | 63.9 | 0.17 | 550 | 1060 |
| 5 | 63.9 | 0.25 | 550 | 1152 |
| 6 | 69.0 | 0.33 | 336 | 570 |
| 7 | 69.0 | 0.33 | 294 | 498 |
| 8 | 66.0 | 0.33 | 500 | 737 |

4

It can be seen from the above results that all these compositions retained their gel structure at temperatures up to 36°C. Also when each of the above compositions was dispensed through the nozzle of a syringe on to the palm of the technician's hand the gel structure reformed rapidly giving a creamy product with a pleasant feel.

### EXAMPLE 9 (comparative)

The experiment described in Example 1 was repeated except that the hydrophilic polymer was replaced by an identical quantity of a different, medium molecular weight, sodium carboxymethyl cellulose which was sold under the trade name "BLANOSE 7MX". This polymer had a viscosity in a 2% by weight aqueous suspensions at 22°C of more than 100 mPas when measured by Brookfield Viscometer with a spindle speed of 60 rpm. It was found that the polymer was insufficiently soluble in water to remain in solution when the isopropanol was added and it was impossible to form a stable gel in the final composition.

**Claims**

1. A stable, thixotropic, fluid composition which comprises water, a lower aliphatic alcohol, a hydrophilic smectite clay and a relatively low molecular weight hydrophilic polymer, wherein the ratio by weight of lower aliphatic alcohol to water is in the range 1:4 to 7:3, the ratio by weight of clay to water is in the range of from 1:4 to 1:19; and the ratio by weight of hydrophilic polymer to smectite clay is in the range of from 0.05:1 to 0.5:1.

2. A composition according to Claim 1, wherein the hydrophilic polymer is sodium carboxymethyl cellulose.

3. A composition according to Claim 1 or 2, wherein the weight ratio of the hydrophilic polymer to the dry smectite clay is in the range of from 0.1:1 to 0.4:1.

4. A composition according to Claim 1, 2 or 3, wherein the lower aliphatic alcohol is isopropanol.

5. A method of preparing a composition which consists predominantly of a mixture of a lower aliphatic alcohol and water in a ratio of from 1:4 to 7:3, the method comprising the following steps:-

a) mixing a hydrophilic smectite clay mineral with water to form a viscous suspension in which the ratio of clay to water is in the range of from 1:4 to 1:19;

b) mixing with the viscous suspension a hydrophilic polymer of relatively low molecular weight in a quantity such that the weight ratio of hydrophilic polymer to dry smectite clay mineral is in the range of from 0.05:1 to 0.5:1; and

c) mixing with the suspension formed in step b) a lower aliphatic alcohol.

6. A method according to claim 5, wherein the quantity of the smectite clay used is in the range of from 5% to 20% by weight of the clay-water mixture.

7. A method according to claim 5 or 6, wherein the hydrophilic polymer is sodium carboxymethyl cellulose.

8. A method according to claim 5, 6 or 7, wherein the weight ratio of the hydrophilic polymer to the dry smectite clay is in the range from 0.1:1 to 0.4:1.

9. A method according to any one of claims 5 to 8, wherein the alcohol constitutes from about 20% to about 70% by weight of the alcohol-water mixture.

10. A method according to any one of claims 5 to 9, wherein in step c) the mixture of the suspension formed in step b) and the lower aliphatic alcohol is subjected to high shear mixing.

11. A method according to any one of claims 5 to 10, wherein the lower aliphatic alcohol is isopropanol.

12. A base composition having a creamy texture and which is for use as, or in the preparation of, personal care and toiletry creams and lotions, which comprises water, a lower aliphatic alcohol, a hydrophilic smectite clay and a relatively low molecular weight hydrophilic polymer, wherein the ratio by weight of lower aliphatic alcohol to water is in the range 1:4 to 7:3, the ratio by weight of clay to water is in the range of from 1:4 to 1:19; and the ratio by weight of hydrophilic polymer to smectite clay is in the range of from 0.05:1 to 0.5:1.